# EUROPÄISCHE PATENTSCHRIFT

(11) **EP 2 555 666 B1**
(45) Veröffentlichungstag und Bekanntmachung des Hinweises auf die Patenterteilung: **08.04.2020**
(21) Anmeldenummer: 11706524.3
(22) Anmeldetag: 24.02.2011
(51) Int. Cl.: A61B 1/05, A61B 5/06, A61B 1/005

(54) **ENDOSKOP**
ENDOSCOPE
ENDOSCOPE

(30) Priorität: 09.04.2010 DE 102010003808
(43) Veröffentlichungstag der Anmeldung: 13.02.2013
(73) Patentinhaber: Siemens Healthcare GmbH, 91052 Erlangen (DE)
(72) Erfinder: JULOSKI, Aleksandar, 90491 Nuernberg (DE); BECHTOLD, Mario, 91334 Hemhofen (DE); KELLER, Henrik, 91054 Erlangen (DE)
(86) Internationale Anmeldenummer: PCT/EP2011/052747
(87) Internationale Veröffentlichungsnummer: WO 2011/124414

(56) Entgegenhaltungen:
- WO-A1-2009/056441
- GB-A- 2 352 636
- JP-A- 2003 260 026
- US-A- 5 966 168
- US-A1- 2003 125 788
- US-A1- 2003 181 788
- US-A1- 2005 216 231
- US-A1- 2005 272 976
- US-A1- 2009 005 645

## Beschreibung

Die Erfindung betrifft eine medizinische Vorrichtung, umfassend ein System zum Erzeugen eines externen Magnetfelds und Endoskop mit einem Endoskopkopf, der in einen Hohlraum einführbar ist, und einem Endoskopschlauch, der mit dem Endoskopkopf verbunden ist.

Ein derartiges Endoskop dient zur Untersuchung des Gastrointestinaltrakts und umfasst einen Endoskopiekopf, an dem ein schubsteifer, flexibler Endoskopschlauch befestigt ist. Das Endoskop wird während einer Untersuchung oral (Gastroskopie) oder rektal eingeführt (Koloskopie, Rektoskopie) und im Gastrointestinaltrakt vorgeschoben. Nachteilig hierbei ist, dass der Endoskopschlauch relativ steif ist, da über ihn die Vorschubkraft eingeleitet werden muss. Ein derartiges Vorwärtsschieben des Endoskopkopfs bedeutet, dass von der Körperöffnung weiter entfernte Gebiete schwer oder gar nicht erreichbar sind. Für den Arzt ist die manuelle Bedienung des Endoskops relativ aufwendig und kompliziert, da er ein zu starkes Drücken des Endoskopkopfs gegen die Organwände zu vermeiden versucht. Trotzdem ist diese Art der Endoskopie für den Patienten relativ unangenehm.

Eine Alternative zu einer mittels eines flexiblen Endoskops durchgeführten Gastroskopie besteht in der Verwendung einer sogenannten Endoskopiekapsel. Eine derartige Endoskopiekapsel, die auch als Kapselendoskop oder Endokapsel bezeichnet wird, ist als passive Endokapsel oder als navigierbare Endokapsel ausgeführt. Eine passive Endoskopiekapsel bewegt sich aufgrund der Peristaltik durch den Darm des Patienten.

Eine navigierbare Endoskopiekapsel ist beispielsweise aus dem Patent mit der Veröffentlichungsnummer DE 101 42 253 C1 sowie aus der korrespondierenden Patentanmeldung mit der Veröffentlichungsnummer US 2003/0060702 A1 bekannt und wird dort als "Endoroboter" bzw. "endo-robot" bezeichnet. Der aus diesen Veröffentlichungen bekannte Endoroboter kann mittels eines Magnetfelds, das von einem externen (d.h. außerhalb des Patienten angeordneten) Magnetsystem (Spulensystem) erzeugt wird, in einem Hohlorgan (z.B. Magen-Darm-Trakt) eines Patienten navigiert werden. Über ein integriertes System zur Lagekontrolle, das eine Positionsmessung des Endoroboters und eine automatische Regelung des Magnetfelds bzw. der Spulenströme umfasst, können automatisch Änderungen der Lage des Endoroboters im Hohlorgan des Patienten erkannt und kompensiert werden. Weiterhin kann der Endoroboter gezielt in gewünschte Regionen des Hohlorgans navigiert werden. Diese Art der Kapselendoskopie wird deshalb auch als MGCE (Magnetically Guided Capsule Endoscopy - magnetisch geführte Kapselendoskopie) bezeichnet.

Die Endoskopiekapsel gemäß dem Patent DE 101 42 253 C1 ist sowohl für eine Koloskopie (endoskopisch durchgeführte Untersuchung des menschlichen oder tierischen Darms) als auch für eine Gastroskopie (endoskopisch durchgeführte Untersuchung des menschlichen oder tierischen Magens) geeignet.

Bei einer Gastroskopie wird die Endoskopiekapsel dem Patienten oral verabreicht und gelangt über die Speiseröhre in den Magen. Während der Gastroskopie werden verschiedene Größen, Messwerte oder Proben im Inneren des Magens aufgenommen und einem Arzt oder Assistenten zur Auswertung zur Verfügung gestellt. Beispielsweise werden Inhaltsstoffe oder Konzentrationen des Mageninhalts gemessen, die chemische Zusammensetzung des Magensafts bestimmt oder Bilddaten von der Magenschleimhaut gesammelt.

Zur Übertragung von Messdaten aus dem Inneren des Magens steht die Endoskopiekapsel z.B. über eine Funkverbindung mit einer in der Nähe des Patienten aufgestellten Übertragungsstation in Verbindung. Zur gezielten Aufnahme von Mess- und/oder Bilddaten aus bestimmten Regionen des Magens ist die Endoskopiekapsel entsprechend magnetisch navigierbar. Im Gegensatz zur konventionellen Gastroskopie wird der Magen bei der Kapselendoskopie nicht mit einem Gas, sondern mithilfe einer zugeführten Flüssigkeit (Trinklösung, z.B. Wasser) aufgeweitet, die wahlweise mit einer Magensonde in den Magen des Patienten verbracht wird oder die dem Patienten zur selbstständigen Aufnahme verabreicht wird.

Die Endoskopiekapsel weist ein biokompatibles Gehäuse auf, in dem wenigstens ein Magnetelement zur Navigation mittels eines von einem externen Magnetsystem erzeugbaren Magnetfelds sowie wenigstens eine Sensoreinrichtung zur Erfassung medizinisch relevanter Daten und/oder wenigstens eine Therapievorrichtung zur Verabreichung eines Therapiemittels angeordnet sind.

Das Gehäuse der Endoskopiekapsel ist beispielsweise entweder ellipsoidförmig oder zylinderförmig ausgebildet. Ein zylinderförmig ausgebildetes Gehäuse weist in wenigstens einem seiner beiden stirnseitigen Bereiche eine halbsphärische Kappe auf. Vorzugsweise weisen beide stirnseitigen Bereiche des Gehäuses jeweils eine halbsphärische Kappe aus einem optisch transparenten Material auf. Eine derartige Endoskopiekapsel kann dann an beiden stirnseitigen Bereichen jeweils eine optische Sensoreinrichtung (z.B. CMOS-Kamera oder CCD-Chip) aufweisen.

Weiterhin ist aus der Patentanmeldung mit der Veröffentlichungsnummer DE 10 2005 032 368 A1 eine Endoskopiekapsel bekannt, die wenigstens ein mit einem externen Magnetfeld wechselwirkendes magnetisches Element enthält, das zur magnetischen Navigation der Endoskopiekapsel dient. An der Endoskopiekapsel ist ein aus einem flexiblen und nicht schubsteifen Material bestehender Schlauch angeordnet, über den der Endoskopiekapsel flüssige oder gasförmige Betriebs- oder Arbeitsmittel zuführbar sind. Alternativ oder zusätzlich ist in dem Schlauch wenigstens eine Leitung zur Endoskopiekapsel geführt, die der Signal- oder Stromleitung dient. Die bekannte Endoskopiekapsel benötigt für ihre translatorische Bewegung ein relativ starkes Magnetfeld, so dass eine gezielte Navigation innerhalb des Gastrointestinaltrakts nur entsprechend schwierig beherrschbar ist.

Die US 2005/0272976 A1 beschreibt eine Hilfsvorrichtung für die Endoskop-Einführung, wobei die Hilfsvorrichtung ein flexibles Rohr und ein distales Endelement umfasst. Das distale Endelement ist an dem distalen Ende des Rohrs angeordnet, wobei der Außendurchmesser des distalen Endelements größer ist als der Außendurchmesser des Rohrs. Ferner umfasst die Hilfsvorrichtung eine Spiralstruktur, die an der Außenumfangsfläche des Rohrs und an der Außenumfangsfläche des distalen Endelements angeordnet ist. Weiterhin weist die bekannte Hilfsvorrichtung ein Rotationsantriebsmittel auf, das das Rohr drehbar antreibt. Die Rotationsantriebsmittel umfassen z.B. einen Motor, der einen Kompressor antreibt, der mit einer Luftzuführungseinrichtung gekoppelt ist. Alternativ treibt der Motor eine Magnetanordnung an. Das Magnetfeld, durch das das flexible Rohr antreibbar ist, wird innerhalb der Hilfsvorrichtung durch eine motorische Bewegung von Permanentmagneten erzeugt. Somit tritt nur eine Wechselwirkung des flexiblen Rohres mit einem intern (d.h. innerhalb der Endoskopeinführungs-Hilfsvorrichtung) erzeugten Magnetfeld auf. Das Rohr muss also schubsteif sein und kann somit nicht als Endoskopschlauch ausgeführt sein.

Weiterhin ist aus der US 2005/0216231 A1 ein System zur Detektion der Position und der Stellung eines kapselförmigen Medizinprodukts bekannt. Bei dem Medizinprodukt handelt sich um eine magnetisch geführte Endoskopiekapsel, die bei einer MGCE (Magnetically Guided Capsule Endoscopy - magnetisch geführte Kapselendoskopie) verwendet wird.

Aufgabe der vorliegenden Erfindung ist es, eine medizinische Vorrichtung umfassend ein Endoskop zu schaffen, das von einem Benutzer auf einfache Weise innerhalb eines Gastrointestinaltrakts verschiebbar ist.

Die Aufgabe wird erfindungsgemäß durch ein Endoskop nach Anspruch 1 gelöst. Vorteilhafte Ausgestaltungen des erfindungsgemäßen Endoskops sind jeweils Gegenstand von weiteren Ansprüchen.

Die medizinische Vorrichtung nach Anspruch 1 umfasst ein System zum Erzeugen eines externen Magnetfelds und ein Endoskopkopf, der in einen Hohlraum (z.B. Gastrointestinaltrakt eines Patienten) einführbar ist, und einen Endoskopschlauch, der mit dem Endoskopkopf verbunden ist. Erfindungsgemäß umfasst der Endoskopkopf ein Gehäuse, an dessen Außenwand zumindest in einem Teilbereich ein Gewinde angeordnet ist, und wenigstens ein Magnetelement, das mit einem externen Magnetfeld wechselwirkt, sowie wenigstens eine Kamera, die vom Inneren des Hohlraums Aufnahmen liefert.

Dadurch, dass bei dem erfindungsgemäßen Endoskop der Endoskopkopf an der Außenfläche des Gehäuses zumindest in einem Teilbereich ein Gewinde aufweist, und dadurch, dass der Endoskopkopf wenigstens ein Magnetelement umfasst, kann der Endoskopkopf durch ein extern erzeugtes magnetisches Grundfeld, das magnetische Kräfte auf das Magnetelement bzw. auf die Magnetelemente ausübt, in Rotationsbewegungen (Schraubbewegungen) versetzt werden.

Durch Generierung externer Magnetfelder kann so ein Drehmoment erzeugt werden, das das Magnetelement und damit den Endoskopkopf in eine Rotation um seine Längsachse versetzt, so dass der Endoskopkopf durch Schraubbewegungen vorwärts oder rückwärts durch den Darm bewegt werden kann.

Bei einer Schraubbewegung dreht sich der Endoskopkopf gleichzeitig um eine Gerade (Schraubachse) und wird entlang dieser Geraden verschoben.

Da das für die magnetische Navigation des Endoskopkopfs extern erzeugte magnetische Grundfeld den größten Wirkungsgrad für Rotationen aufweist, ist für eine Schraubbewegung des Endoskopkopfs nur ein entsprechend geringes Magnetfeld erforderlich, das mit einem entsprechend geringen Energieverbrauch realisierbar ist.

Durch das erfindungsgemäß vorgesehene Gewinde werden Vor- und Rückwärtsbewegungen des Endoskopkopfs mithilfe von Rotationen erzeugt. Ein Gleiten des Endoskopkopfs an den Innenwänden des Gastrointestinaltrakts wird bei der erfindungsgemäßen Lösung durch eine Schraubbewegung des Endoskopkopfs ersetzt.

Das erfindungsgemäße Endoskop ist dadurch auf einfache Weise magnetisch navigierbar. Gegenüber einer reinen Linearbewegung, die durch manuell aufgebrachte Zug- und Schubkräfte erzeugt wird, ist bei der erfindungsgemäßen Lösung für die Fortbewegung des Endoskopiekopfs ein deutlich geringerer Kraftaufwand erforderlich. Da keine manuelle Krafteinleitung auf den Endoskopiekopf vorgenommen wird, muss der Endoskopschlauch demzufolge auch nicht als schubsteifer Endoskopschlauch ausführt sein. Für den Patienten ist eine Endoskopie mit dem erfindungsgemäßen Endoskop somit wesentlich weniger belastend. Darüber hinaus wird die Bedienbarkeit des Endoskops vereinfacht, so dass auch von der Körperöffnung weiter entfernte Gebiete leichter oder überhaupt erst erreichbar sind. Das Endoskop nach Anspruch 1 ist damit gleichermaßen gut für Gastroskopien sowie für Koloskopien und für Rektoskopien geeignet.

Das Magnetelement kann im Rahmen der Erfindung innerhalb des Gehäuses des Endoskopskopfs angeordnet sein. Gemäß einer besonders bevorzugten Ausgestaltung nach Anspruch 2 verläuft das Gewinde zumindest teilweise in der Außenwand des Gehäuses und ist als umlaufende Nut bzw. als umlaufende Rille ausgebildet. Das nutförmige bzw. rillenförmige Gewinde ist dadurch auf konstruktiv einfache Weise in die Außenwand des Gehäuses integriert.

Eine ebenfalls vorteilhafte Ausgestaltung gemäß Anspruch 3 ist dadurch gekennzeichnet, dass das Gewinde zumindest teilweise auf der Außenwand des Gehäuses verläuft und als umlaufende Wulst ausgebildet ist. Das wulstförmige, an der der Außenwand des Gehäuses anliegende Gewinde ist ebenfalls auf konstruktiv einfache Weise realisierbar.

Bei einer besonders bevorzugten Ausführungsform nach Anspruch 4 ist das Gehäuse zumindest teilweise als Magnetelement ausgebildet. In Verbindung mit einer Integration des Gewindes in der Außenwand des Gehäuses - wie in den Ansprüchen 2 und 3 beschrieben - ist das Gehäuse des Endoskopkopfs sowie das Gewinde und das Magnetelement als gemeinsames Bauteil ausgeführt. Dadurch ergibt sich für die Herstellung des Endoskops eine Reduzierung der Montageschritte.

Die Ausführungsform nach Anspruch 5 ist dadurch gekennzeichnet, dass wenigstens ein Magnetelement zumindest teilweise auf der Außenwand des Gehäuses angeordnet ist, wobei gemäß einer besonders vorteilhaften Ausgestaltung nach Anspruch 6 das Magnetelement das Gehäuse ringförmig umschließt. Ebenfalls vorteilhaft ist eine alternative Ausgestaltung zu dem Endoskop gemäß Anspruch 6, die in Anspruch 7 beschrieben ist. Bei der Ausführungsform nach Anspruch 7 ist das Magnetelement ringförmig ausgebildet und vom Gehäuse umschlossen.

In vorteilhafter Weise ist bei einer Ausführungsform gemäß Anspruch 8 die Kamera rotatorisch vom Gehäuse des Endoskopkopfs entkoppelt. Dadurch führt die Kamera im Wesentlichen nur Rotationsbewegungen aus, die vom Arzt, der die Endoskopie durchführt, auch gewollt sind. Durch die weitgehende Vermeidung ungewollter Rotationsbewegungen der Kamera wird die Bildgebung deutlich verbessert, was die Navigation für den endoskopierenden Arzt deutlich erleichtert.

Gemäß Erfindung ist der Endoskopschlauch rotatorisch vom Gehäuse des Endoskopkopfs entkoppelt. Bei einer Schraubbewegung des Endoskopkopfs, die eine translatorische Bewegung des Endoskops bewirkt, führt der Endoskopschlauch somit keine Rotationsbewegungen aus. Für den Patienten ist damit die endoskopische Untersuchung mit einem derartig ausgestalteten Endoskop deutlich angenehmer.

Im Rahmen der Erfindung ist auch eine gemeinsame rotatorische Entkopplung von Kamera und Endoskopschlauch gegenüber dem Gehäuse realisierbar. Für eine vorteilhafte Realisierung können z.B. die Kamera und der Endoskopschlauch verdrehfest miteinander gekoppelt und das Gehäuse des Endoskopkopfs und/oder Magnetelement auf dem Endoskopschlauch drehbar gelagert sein.

Weiterhin sind für das Gehäuse des Endoskopskopfs verschiedene Formen realisierbar. So ist beispielsweise ein (abgeschnittener) Ellipsoid oder ein Zylinder möglich. Bei der Optimierung der Größe des Gehäuses des Endoskopkopfs ist zu berücksichtigen, dass ein Gehäuse mit einem größeren Durchmesser einerseits den Widerstand für den Vortrieb des Endoskopskopfs vergrößert, andererseits jedoch die umliegenden Darmwände bei einem Endoskopkopf mit einem größeren Durchmesse stärker gedehnt werden, was eine bessere Sicht schafft.

Die Erfindung sowie weitere vorteilhafte Ausgestaltungen werden im Folgenden anhand von schematisch dargestellten Ausführungsbeispielen in der Zeichnung näher erläutert, ohne jedoch auf die erläuterten Ausführungsbeispiele beschränkt zu sein.

Es zeigen:
- FIG 1: einen Längsschnitt durch eine erste Ausführungsform eines erfindungsgemäßen Endoskops,
- FIG 2: eine Seitenansicht des Endoskops gemäß FIG 1,
- FIG 3: eine Seitenansicht einer zweiten Ausführungsform eines erfindungsgemäßen Endoskops,
- FIG 4: eine Seitenansicht einer dritten Ausführungsform eines erfindungsgemäßen Endoskops,
- FIG 5: eine Seitenansicht einer vierten Ausführungsform eines erfindungsgemäßen Endoskops,
- FIG 6: eine Seitenansicht einer fünften Ausführungsform eines erfindungsgemäßen Endoskops.

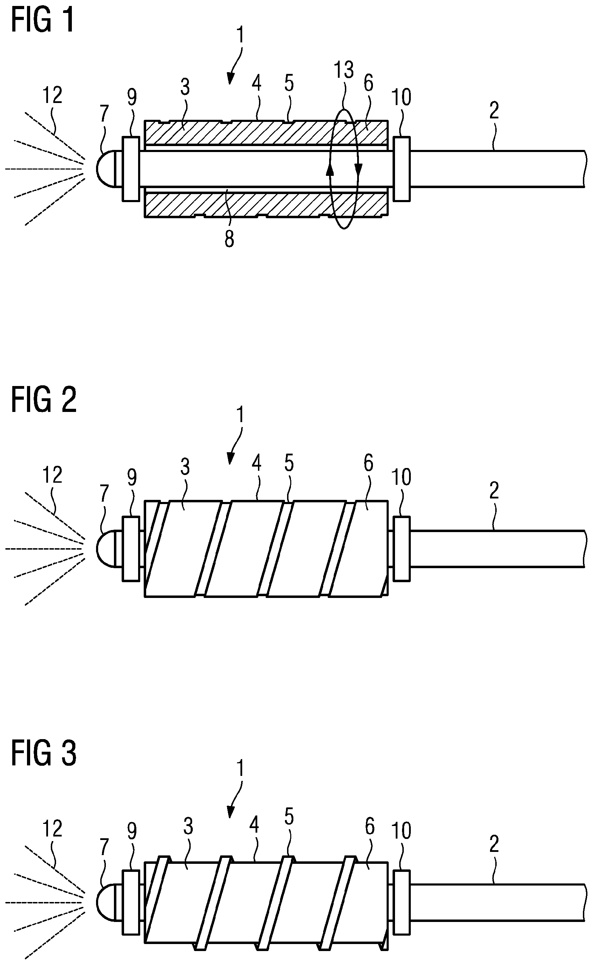

Bei den in den FIG 1 bis 6 dargestellten Ausführungsbeispielen des erfindungsgemäßen Endoskops ist jeweils mit 1 ein Endoskopkopf bezeichnet. Der Endoskopkopf 1 ist in einen Hohlraum, z.B. in einen Gastrointestinaltrakt eines Patienten, einführbar.

Jedes in den FIG 1 bis 6 dargestellte Endoskop 1 umfasst weiterhin einen Endoskopschlauch 2, der mit dem Endoskopkopf 1 verbunden ist. Erfindungsgemäß umfasst der Endoskopkopf 1 ein Gehäuse 3, an dessen Außenwand 4 zumindest in einem Teilbereich ein Gewinde 5 angeordnet ist, und wenigstens ein Magnetelement 6, das mit einem externen Magnetfeld wechselwirkt, sowie wenigstens eine Kamera 7, die vom Inneren des Hohlraums Aufnahmen liefert. Das Blickfeld der Kamera 7 ist jeweils mit 12 bezeichnet.

Dadurch, dass bei den in den FIG 1 bis 6 gezeigten Endoskopen jeweils der Endoskopkopf 1 an der Außenfläche des Gehäuses 3 zumindest in einem Teilbereich ein Gewinde 5 aufweist, und dadurch, dass der Endoskopkopf 1 jeweils wenigstens ein Magnetelement 6 umfasst, kann der Endoskopkopf 1 durch ein extern erzeugtes magnetisches Grundfeld, das magnetische Kräfte auf das Magnetelement 6 bzw. auf die Magnetelemente 6 ausübt, in eine Rotationsbewegung (Schraubbewegung) versetzt werden. Die Rotationsbewegung (Schraubbewegung) des Endoskopkopfs 1 ist in FIG 1 mit 13 bezeichnet.

Bei der Ausführungsform gemäß FIG 1 und 2 weist das Gehäuse 3 des Endoskopiekopfs 1 eine Zylinderform auf. Das für die Rotationsbewegung des Endoskopkopfs 1 notwendige Gewinde 5 verläuft in der Außenwand 4 des Gehäuses 3 und ist als umlaufende Nut bzw. umlaufende Rille ausgeführt. Das Gewinde 5 ist somit in der Außenwand 4 des Gehäuses 3 integriert. Das Magnetelement 6 ist bei der in FIG 1 und 2 gezeigten Ausgestaltung im Gehäuse 3 des Endoskopkopfs 1 integriert bzw. wird vom Gehäuse 3 gebildet.

Weiterhin ist das Gehäuse 3 mit einer Durchgangsbohrung 8 versehen, durch die der Endoskopschlauch 2 eingeführt ist. Der Innendurchmesser der Durchgangsbohrung 8 ist etwas größer als der Außendurchmesser des Endoskopschlauchs 2, so dass das Gehäuse 3 des Endoskopkopfs 1 gegenüber dem Endoskopschlauch 2 drehbeweglich ist. Der Endoskopschlauch 2 ist damit vom Gehäuse 3 des Endoskopkopfs 1 rotatorisch entkoppelt.

Um eine axiale Fixierung des Endoskopkopfs 1 auf bzw. gegenüber dem Endoskopsschlauch 2 zu erhalten, ist an beiden Stirnseiten des Gehäuses 3 jeweils ein Fixierelement 9 bzw. 10 angeordnet.

An der dem Endoskopschlauch 2 abgewandten Seite des Endoskopkopfs 1 ist die Kamera 7 angeordnet, so dass sich das Blickfeld 12 der Kamera 7 in die Bewegungsrichtung des Endoskops erstreckt.

Die Kamera 7 ist ebenfalls rotatorisch vom Gehäuse 3 entkoppelt, so dass eine Rotationsbewegung (Schraubbewegung) des Gehäuses 3 das Blickfeld 12 der Kamera 7 nicht negativ beeinflusst.

In den FIG 1 und 2 nicht dargestellt, aber dennoch vorteilhaft ist es, falls nur eine Bildgebung mit dem Endoskop erfolgt, den Endoskopschlauch 2 außerhalb des Gehäuses 3 als Flachkabel und innerhalb des Gehäuses 3 als Rundkabel auszuführen. Der als Flachkabel ausgeführte Teil des Endoskopschlauchs 2 kann so zur Stabilisierung der Kamera 7 gegen die Rotation des Gehäuses 3 beitragen.

Bei der in FIG 3 gezeigten Ausführungsform ist das Gewinde 5 nicht in die Außenwand 4 des Gehäuses 3 integriert, wie bei der Ausgestaltung gemäß den FIG 1 und 2, sondern als umlaufende Wulst auf der Außenwand 4 des Gehäuses 3 angeordnet. Damit kann das Gewinde 5 beispielsweise aus einem anderen Material bestehen als das Gehäuse 3, das gleichzeitig das Magnetelement 6 bildet. Der sonstige Aufbau des Endoskops nach FIG 3 entspricht dem Aufbau des in den FIG 1 und 2 dargestellten Endoskops.

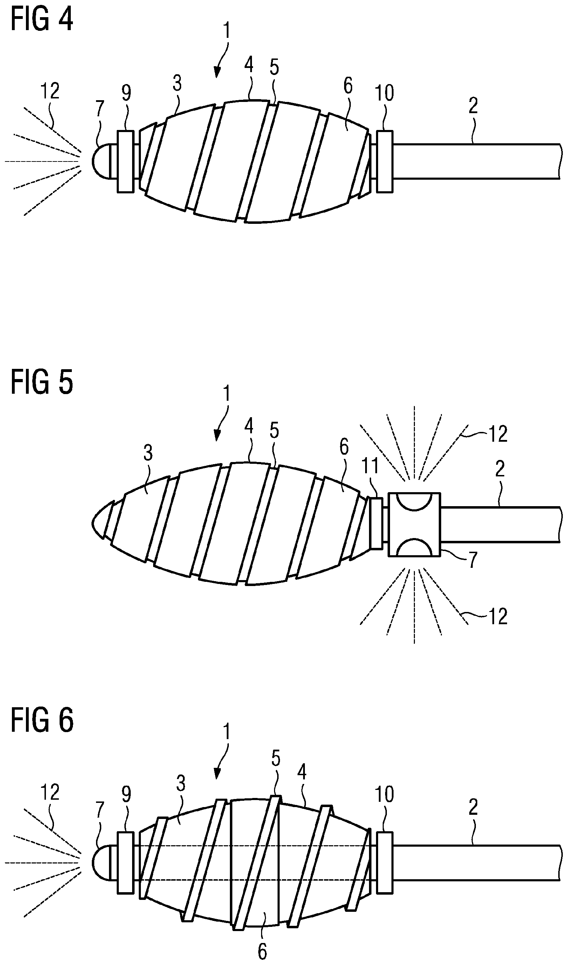

Die in FIG 4 dargestellte Ausgestaltung weist ein Gehäuse 3 auf, bei dem das Magnetelement 6 wiederum vom Gehäuse 3 des Endoskopkopfs 1 gebildet wird. Gegenüber der ersten, in den FIG 1 und 2 gezeigten Ausführungsform weist das Gehäuse 3 eine andere Form auf, nämlich ein an beiden Enden gekapptes Ellipsoid. Alle anderen konstruktiven Merkmale entsprechen dem ersten Ausführungsbeispiel.

Bei dem Ausführungsbeispiel gemäß FIG 5 ist die Kamera 7 dem Gehäuse 3 des Endoskopkopfs 1 nachgelagert und besitzt ein Blickfeld 12, das sich seitlich erstreckt. Weiterhin ist das Magnetelement 6 im Gehäuse 3 des Endoskopkopfs 1 integriert, das Gehäuse 3 bildet somit gleichzeitig das Magnetelement 6. Das Gehäuse 3 ist außerdem auf einem Wälzlager 11 gelagert, so dass eine Rotation des Gehäuses 3 keine Auswirkung auf die Drehstellung der Kamera 7 hat.

In FIG 6 ist eine Ausführungsform dargestellt, bei der das Gehäuse 3 des Endoskopkopfs 1 und das als Magnetring ausgeführte Magnetelement 6 sowie das Gewinde 5 den rotierenden Teil des Endoskops bilden.

Das Magnetelement 6 ist als tragendes Teil ausgeführt und an der Außenwand 4 des Gehäuses 3 fixiert. Alternativ ist es auch möglich, das Magnetelement 6 (Magnetring) an der Innenwand des Gehäuses 3 zu fixieren. Das Gewinde 5 kann hierbei z.B. als weicher Überzug gefertigt sein, der das Gehäuse 3 vollständig umschließt.

Das Blickfeld 12 der Kamera 7 erstreckt sich wiederum in die Bewegungsrichtung des Endoskops.

## Patentansprüche

1. Medizinische Vorrichtung mit einem System zum Erzeugen eines externen Magnetfelds und einem Endoskop, das zur magnetischen Navigation durch das externe Magnetfeld ausgebildet ist, **dadurch gekennzeichnet, dass** das Endoskop mit einem Endoskopkopf (1), der in einen Hohlraum einführbar ist, und mit einem Endoskopschlauch (2), der mit dem Endoskopkopf (1) verbunden ist, ausgebildet ist, wobei der Endoskopkopf (1)
- ein Gehäuse (3), an dessen Außenwand (4) zumindest in einem Teilbereich ein Gewinde (5) zum Erzeugen von Vor- und Rückwärtsbewegungen des Endoskopkopfes (1) mithilfe von Rotationen angeordnet ist, und
- wenigstens eine Kamera (7), die vom Inneren des Hohlraums Aufnahmen liefert, umfasst,
wobei am Endoskopkopf (1) wenigstens ein Magnetelement (6) zum Erzeugen eines Drehmomentes auf den Endoskopkopf (1) durch Wechselwirkung mit dem externen Magnetfeld vorgesehen ist und der Endoskopschlauch (2) vom Gehäuse (3) rotatorisch entkoppelt ist.

2. Medizinische Vorrichtung nach Anspruch 1, **dadurch gekennzeichnet, dass** das Gewinde (5) zumindest teilweise in der Außenwand (4) des Gehäuses (3) verläuft und als umlaufende Nut bzw. als umlaufende Rille ausgebildet ist.

3. Medizinische Vorrichtung nach Anspruch 1 oder 2, **dadurch gekennzeichnet, dass** das Gewinde (5) zumindest teilweise auf der Außenwand (4) des Gehäuses (3) verläuft und als umlaufende Wulst ausgebildet ist.

4. Medizinische Vorrichtung nach einem der Ansprüche 1 bis 3, **dadurch gekennzeichnet, dass** das Gehäuse (3) zumindest teilweise als Magnetelement (6) ausgebildet ist.

5. Medizinische Vorrichtung nach einem der Ansprüche 1 bis 3, **dadurch gekennzeichnet, dass** wenigstens ein Magnetelement (6) zumindest teilweise auf der Außenwand (4) des Gehäuses (3) angeordnet ist.

6. Medizinische Vorrichtung nach Anspruch 5, **dadurch gekennzeichnet, dass** das Magnetelement (6) ringförmig ausgebildet ist und das Gehäuse (3) umschließt.

7. Medizinische Vorrichtung nach einem der Ansprüche 1 bis 3, **dadurch gekennzeichnet, dass** das Magnetelement (6) ringförmig ausgebildet und vom Gehäuse (3) umschlossen ist.

8. Medizinische Vorrichtung nach einem der Ansprüche 1 bis 7, **dadurch gekennzeichnet, dass** die Kamera (7) vom Gehäuse (3) rotatorisch entkoppelt ist.

## Claims

1. Medical apparatus having a system for generating an external magnetic field and an endoscope which is embodied for magnetic navigation through the external magnetic field, **characterised in that** the endoscope is embodied with an endoscope head (1) which can be inserted into a cavity, and with an endoscope tube (2) which is connected to the endoscope head (1), wherein the endoscope head (1) comprises
- a housing (3), on the outer wall (4) of which a thread (5) is arranged at least in a partial area for generating forward and backward movements of the endoscope head (1) with the help of rotations, and
- at least one camera (7) which delivers images from the interior of the cavity,
wherein at least one magnetic element (6) for generating a torque onto the endoscope head (1) by interaction with an external magnetic field is provided on the endoscope head (1) and the endoscope tube (2) is rotationally decoupled from the housing (3).

2. Medical apparatus according to claim 1, **characterised in that** the thread (5) runs at least partially in the outer wall (4) of the housing (3) and is embodied as a circumferential groove or as a circumferential indentation.

3. Medical apparatus according to claim 1 or 2, **characterised in that** the thread (5) runs at least partially on the outer wall (4) of the housing (3) and is embodied as a circumferential bead.

4. Medical apparatus according to one of claims 1 to 3, **characterised in that** the housing (3) is at least partially embodied as a magnetic element (6).

5. Medical apparatus according to one of claims 1 to 3, **characterised in that** at least one magnetic element (6) is at least partially arranged on the outer wall (4) of the housing (3) .

6. Medical apparatus according to claim 5, **characterised in that** the magnetic element (6) is embodied as annular and encloses the housing (3).

7. Medical apparatus according to one of claims 1 to 3, **characterised in that** the magnetic element (6) is embodied as annular and is enclosed by the housing (3).

8. Medical apparatus according to one of claims 1 to 7, **characterised in that** the camera (7) is rotationally decoupled from the housing (3).

## Revendications

1. Dispositif médical, comprenant un système de production d'un champ magnétique extérieur et un endoscope, constitué pour la navigation magnétique dans le champ magnétique extérieur, **caractérisé en ce que** l'endoscope est constitué en ayant une tête (1) d'endoscope, qui peut être introduite dans une cavité, et en ayant un tube souple (2) d'endoscope, qui peut être relié à la tête (1) d'endoscope, la tête (1) d'endoscope comprenant
- un boîtier (3) sur la paroi (4) extérieure duquel est disposé, au moins dans une région partielle, un filetage (5) pour produire des déplacements d'aller et retour de la tête (1) de l'endoscope au moyen de rotations, et
- au moins une caméra (7), qui fournit des enregistrements de l'intérieur de la cavité,
dans lequel il est prévu, à la tête (1) de l'endoscope, au moins un élément (6) magnétique pour produire un couple sur la tête (1) de l'endoscope, par interaction avec le champ magnétique extérieur, et le tube (2) souple de l'endoscope est découplé en rotation du boîtier (3).

2. Dispositif médical suivant la revendication 1, **caractérisé en ce que** le filetage (5) s'étend, au moins en partie, dans la paroi (4) extérieure du boîtier (3) et est constitué sous la forme d'une encoche faisant le tour ou comme une rainure faisant le tour.

3. Dispositif médical suivant l'une des revendications 1 à 2, **caractérisé en ce que** le filetage (5) s'étend, au moins en partie, sur la paroi (4) extérieure du boîtier (3) et est constitué sous la forme d'un bourrelet faisant le tour.

4. Dispositif médical suivant l'une des revendications 1 à 3, **caractérisé en ce que** le boîtier (3) est constitué, au moins en partie, sous la forme d'un élément (6) magnétique.

5. Dispositif médical suivant l'une des revendications 1 à 3, **caractérisé en ce qu'**au moins un élément (6) magnétique est disposé, au moins en partie, sur la paroi (4) extérieure du boîtier (3).

6. Dispositif médical suivant la revendication 5, **caractérisé en ce que** l'élément (6) magnétique est constitué annulairement et entoure le boîtier (3).

7. Dispositif médical suivant l'une des revendications 1 à 3, **caractérisé en ce que** l'élément (6) magnétique est constitué annulairement et est entouré du boîtier (3).

8. Dispositif médical suivant l'une des revendications 1 à 7, **caractérisé en ce que** la caméra (7) est découplée en rotation du boîtier (3).
